# EUROPEAN PATENT APPLICATION

(11) **EP 4 755 919 A1**
(43) Date of publication of application: **10.06.2026**
(21) Application number: 23862084.3
(22) Date of filing: 28.07.2023
(51) Int. Cl.: C07K 16/30, C12N 15/13, C12N 15/85, C12N 5/10, A61K 39/395, A61P 35/00

(54) **ANTI-TROP2 ANTIBODY, CONJUGATE COMPRISING SAID ANTIBODY, AND USE THEREOF**

(71) Applicant: Hanano Technologies Limited, Prestbury, England GL52 3DG (GB)
(72) Inventor: WAN, Yakun, Prestbury, England GL52 3DG (GB); ZHU, Min, Prestbury, England GL52 3DG (GB); GAI, Junwei, Prestbury, England GL52 3DG (GB); LI, Guanghui, Prestbury, England GL52 3DG (GB); BISHOP, Benjamin, Prestbury, Chelteham Gloucestershire (GB)
(74) Representative: Mewburn Ellis LLP
(86) International application number: PCT/CN2023/109907
(87) International publication number: WO 2024/051383

(57) **Abstract**

Provided are an anti-Trop2 antibody, a conjugate comprising said antibody, and a use thereof. Specifically, provided are an anti-Trop2 single-domain antibody, a long-acting anti-Trop2 antibody formed from connecting, in tandem, an anti-Trop2 single domain antibody and an anti-serum albumin single domain antibody, and an antibody-drug conjugate constructed on the basis of the long-acting anti-Trop2 antibody. Further provided are an encoding sequence encoding the antibody, a corresponding expression vector, and a host cell capable of expressing the antibody. The anti-Trop2 antibody and the conjugate thereof can be used for the treatment or diagnosis of Trop2-related diseases.

## Description

### TECHNICAL FIELD

The present invention relates to the technical field of biomedicine or biopharmaceuticals, in particular to an anti-Trop2 antibody, conjugate comprising the antibody and appllication thereof.

### BACKGROUND

Trop2 belongs to the TACSTD family, which is a cell surface glycoprotein encoded and expressed by the TACSTD2 gene, and it is also known as tumor-associated calcium signal transducer 2 (TACSTD2), epidermal glycoprotein 1 (EGP-1), gastrointestinal tumor-associated antigen (GA733-1), and surface marker 1 (M1S1). TROP-2 promotes tumor cell growth, proliferation and metastasis mainly by regulating the calcium signaling pathway, cyclin expression and decreasing fibronectin adhesion. Trop2 can also interact with β-catenin in the Wnt signaling cascade, playing a role in the transcription of nuclear oncogenes and cell proliferation.

Studies have shown that Trop2 is highly expressed in tumors such as gastric cancer, cervical cancer, breast cancer, lung cancer, prostate cancer, colon cancer and uterine mucosal serous papillary carcinoma, and it has been confirmed that TROP2 is significantly correlated with tumor metastasis and prognosis, and plays an important role in the occurrence of tumors. Trop2 is a transmembrane protein whose extracellular domain is overexpressed on a variety of tumors, making it a natural candidate for targeted therapy development. The limitation of tissue expression of Trop2 makes the treatment less toxic, which is also the advantage of Trop2 targeted therapy. Multiple forms of drugs targeting Trop2, including antibodies, antibody conjugates and combination therapy, are under development. The utility of anti-TROP2 antibodies in coupling with other chemotherapy agents has been demonstrated in various preclinical studies. IMMU-132, an antibody-drug conjugate (ADC) for the treatment of TROP2-overexpressing epithelial malignancies, is in phase II/III clinical trials. The new antibody-drug conjugate, Sacituzumab govitecan (IMMU-132), takes Trop2 as the target. It is made by conjugating the humanized antibody hRS7 as the target carrier with the active metabolite SN38 of irinotecan. It can be used to treat a variety of epithelial malignant tumors, such as breast cancer (triple negative breast cancer), ovarian cancer, small cell lung cancer, etc. In addition, other humanized anti-TROP2 IgG-SN-38 conjugates, such as anti-TROP2 hRS7-CL2A-SN-38 antibody-drug conjugate, which has been shown to have significant specific anticancer effects in xenotransplantation models of multiple tumor cell lines (Calu-3, Capan-1, BxPC-3, and COLO-205).

Up to now, nanobody (Nb), known as heavy chain single domain antibody VHH (variable domain of heavy chain of heavy-chain antibody), is a heavy chain antibody (HCAb) naturally lacking light chain in camels, and the single domain antibody consisting of only one heavy chain variable region obtained by cloning its variable region is the currently available smallest unit that can bind to antigen and has stable and full function. Single domain antibodies have the characteristics of high stability, good water solubility, easy for humanization, high targeting ability, and strong penetration, and play a surprisingly huge function in immune experiments, diagnosis and treatment. Single domain antibodies are gradually becoming an emerging force in the diagnosis and treatment by the new generation antibodies.

Therefore, it is necessary to develop an anti-Trop2 single domain antibody in the field, especially an anti-Trop2 single domain antibody with good Trop2 antigen binding ability.

### SUMMARY OF THE INVENTION

The objective of the present invention is to provide an anti-Trop2 antibody, a conjugate comprising the antibody and application thereof.

Specifically, the objective of the present invention is to provide a single domain antibody capable of specifically binding to Trop2 protein, a long-acting anti-Trop2 antibody consisting of the single domain antibody and anti-serum albumin single domain antibody, and conjugates comprising the aforesaid antibodies.

In the first aspect of the present invention, it provides complementary determining regions (CDRs) of the VHH chain of an anti-Trop2 single domain antibody, wherein the complementary determining regions (CDRs) comprise CDR1 as set forth in SEQ ID NO: 6, CDR2 as set forth in SEQ ID NO: 7, and CDR3 as set forth in SEQ ID NO: 8.

In another preferred embodiment, the CDR1, CDR2 and CDR3 are separated by framework regions FR1, FR2, FR3 and FR4.

In the second aspect of the present invention, it provides the VHH chain of an anti-TROP2 single domain antibody, wherein the VHH chain comprises framework regions (FRs) and complementarity determining regions (CDRs) of the first aspect of the present invention.

In another preferred embodiment, the framework regions (FRs) comprise:
(a) FR1 as set forth in SEQ ID NO: 2, F2 as set forth in SEQ ID NO: 3, F3 as set forth in SEQ ID NO: 4, F4 as set forth in SEQ ID NO: 5; or
(b) FR1 as set forth in SEQ ID NO: 10, FR2 as set forth in SEQ ID NO: 11, FR3 as set forth in SEQ ID NO: 12, and FR4 as set forth in SEQ ID NO: 14.

In another preferred embodiment, the VHH chain of the anti-Trop2 single domain antibody is as set forth in SEQ ID NO: 1 or 9.

In the third aspect of the present invention, it provides an anti-Trop2 single domain antibody, wherein the single domain antibody comprises the VHH chain of the second aspect of the present invention.

In another preferred embodiment, the anti-Trop2 single domain antibody comprises a camel-derived single domain antibody, a chimeric single domain antibody, or a humanized single domain antibody.

In another preferred embodiment, the anti-Trop2 single domain antibody comprises monomer, bivalent (bivalent antibody), tetravalent (tetravalent antibody), and/or multivalent (multivalent antibody).

In another preferred embodiment, the anti-Trop2 single domain antibody comprises one or more VHH chains having an amino acid sequence as set forth in SEQ ID NO: 1 or 9.

In another preferred embodiment, the sequence of the VHH chain of the anti-Trop2 single domain antibody is as set forth in SEQ ID NO: 1 and/or 9.

In another preferred embodiment, the anti-Trop2 single domain antibody comprises two VHH chains having amino acid sequences as set forth in SEQ ID NO: 1 or SEQ ID NO: 9.

In another preferred embodiment, the anti-Trop2 single domain antibody comprises four VHH chains having amino acid sequences as set forth in SEQ ID NO: 1 or SEQ ID NO: 9.

In another preferred embodiment, the two VHH chains having amino acid sequences as set forth in SEQ ID NO: 1 or SEQ ID NO: 9 are connected by a linking peptide.

In another preferred embodiment, the four VHH chains having amino acid sequences as set forth in SEQ ID NO: 1 or SEQ ID NO: 9 are connected by linking peptides.

In another preferred embodiment, the linking peptide is selected from the following sequences: (GₐS_{b})ₓ-(GₘSₙ)_{y}, wherein a, b, m, n, x, y = 0 or 1 or 2 or 3 or 4 or 5 or 6 or 7 or 8 or 9 or 10 (preferably, a = 4 and b = 1, m = 3 and n = 1).

In the fourth aspect, it provides a VHH chain of a humanized anti-TROP2 single domain antibody, wherein the amino acid sequence of the VHH chain is as set forth in SEQ ID NO: 14.

In the fifth aspect of the present invention, it provides an anti-Trop2 single domain antibody, wherein the antibody comprises the VHH chain of the fourth aspect of the present invention.

In the sixth aspect of the present invention, it provides a VHH chain of an anti-serum albumin single domain antibody, wherein the complementarity determining regions (CDRs) of the VHH chain comprise CDR1 as set forth in SEQ ID NO: 24, CDR2 as set forth in any one of SEQ ID NO: 25, 29, 30 or 31, and CDR3 as set forth in SEQ ID NO: 26.

In another preferred embodiment, the CDR1, CDR2 and CDR3 are separated by framework regions FR1, FR2, FR3 and FR4.

In another preferred embodiment, the complementarity determining regions (CDRs) of the VHH chain comprise CDR1 as set forth in SEQ ID NO: 24, CDR2 as set forth in SEQ ID NO: 25, and CDR3 as set forth in SEQ ID NO: 26.

In another preferred embodiment, the amino acid sequence of the VHH chain is as set forth in SEQ ID NO: 15.

In another preferred embodiment, the amino acid sequence of the VHH chain is as set forth in any one of SEQ ID NO: 34-36.

In another preferred embodiment, the serum albumin protein comprises human serum albumin.

In the seventh aspect of the present invention, it provides an anti-serum albumin single domain antibody, wherein the antibody comprises the VHH chain of the sixth aspect of the present invention.

In another preferred embodiment, the VHH chain of the antibody is as set forth in SEQ ID NO: 15.

In another preferred embodiment, the amino acid sequence of the VHH chain is as set forth in any one of SEQ ID NO: 34-36.

In the eighth aspect of the present invention, it provides an anti-Trop2 single domain antibody Fc fusion protein, wherein the structure of the fusion protein from N-terminus to C-terminus is as shown in the Formula Ia or Ib:

A-L-B (Ia);

B-L-A (Ib);

wherein,
A is the anti-Trop2 single domain antibody of the third or fifth aspect of the present invention;
B is the Fc fragment of IgG; and
L is none or a flexible linker.

In another preferred embodiment, the flexible linker is a peptide linker

In another preferred embodiment, the peptide linker has 1-50 amino acids, preferably 1-20 amino acids.

In another preferred embodiment, the Fc fragment of IgG includes the Fc fragment of human IgG.

In another preferred embodiment, the peptide linker has a structure of (GGGGS)n, where n is a positive integer of 1-5.

In another preferred embodiment, the Fc fragment of IgG includes the Fc fragment of human IgG.

In another preferred embodiment, the Fc fragment of IgG is selected from the group consisting of Fc fragments of IgG1, IgG2, IgG3, IgG4, and a combination thereof.

In another preferred embodiment, the Fc fragment of IgG is IgG4.

In the ninth aspect of the present invention, it provides a multispecific antibody, wherein the multispecific antibody comprises: the CDR region of the VHH chain of the anti-Trop2 single domain antibody of the first aspect of the present invention, the VHH chain of the anti-Trop2 single domain of the second or fourth aspect of the present invention, the anti-Trop2 single domain antibody of the third or fifth aspect of the present invention, and/or the fusion protein of the eighth aspect of the present invention.

In another preferred embodiment, the multispecific antibody comprises a bispecific antibody, a trispecific antibody, etc.

In the tenth aspect of the present invention, it provides a bivalent antibody, wherein the bivalent antibody comprises the CDR region of the VHH chain of the anti-Trop2 single domain antibody of the first aspect of the present invention, the VHH chain of the anti-Trop2 single domain antibody of the second or fourth aspect of the present invention, the anti-Trop2 single domain antibody of the third or fifth aspect of the present invention, or the fusion protein of the eighth aspect of the present invention.

As used herein, "bivalent antibody" and "bispecific antibody" can be used interchangeably.

In another preferred embodiment, the structure of the bivalent antibody from the N-terminus to the C-terminus is shown in Formula IIa or IIb:

Ab1-P-Ab2 (IIa);

Ab2-P-Ab1 (IIb);

wherein,
Ab1 comprises the CDR region of the VHH chain of the anti-Trop2 single domain antibody of the first aspect of the present invention, or the VHH chain of the anti-Trop2 single domain antibody of the second or fourth aspect of the present invention, or the anti-Trop2 single domain antibody of the third or fifth aspect of the present invention;
Ab2 is the other single domain antibody; and
P is none or a flexible linker.

In another preferred embodiment, the Ab2 comprises anti-serum albumin single domain antibody.

In another preferred embodiment, the Ab2 comprises the VHH chain of anti-serum albumin single domain antibody of the sixth aspect of the present invention, or the anti-serum albumin single domain antibody of the seventh aspect of the present invention.

In another preferred embodiment, the serum albumin comprises human serum albumin.

In another preferred embodiment, the Ab1 has the amino acid sequence as set forth in SEQ ID NO: 9, and Ab2 has the amino acid sequence as set forth in SEQ ID NO: 15.

In another preferred embodiment, the flexible linker is a peptide linker

In another preferred embodiment, the peptide linker has 1-50 amino acids, preferably 1-20 amino acids.

In another preferred embodiment, the peptide linker has a structure of (GGGGS)n, where n is a positive integer of 1-5.

In another preferred embodiment, the peptide linker is (GGGGS)4.

In another preferred embodiment, the amino acid sequence of the bivalent antibody is as set forth in SEQ ID NO: 16.

In the eleventh aspect of the present invention, it provides a polynucleotide encoding a protein selected from the group consisting of the CDR region of the VHH chain of the anti-Trop2 single domain antibody of the first aspect of the present invention, the VHH chain of the anti-Trop2 single domain antibody of the second or fourth aspect of the present invention, the anti-Trop2 single domain antibody of the third or fifth aspect of the present invention, the fusion protein of the eighth aspect of the present invention, the multisepecific antibody of the ninth aspect of the present invention, or the bivalent antibody of the tenth aspect of the present invention.

In another preferred embodiment, the polynucleotide includes DNA or RNA.

In another preferred embodiment, the polynucleotide encodes the bivalent antibody of the ninth aspect of the present invention, and has the nucleotide sequence as set forth in SEQ ID NO: 22.

In the tewlfth aspect of the present invention, it provides an expression vector comprising the polynucleotide of the eleventh aspect of the present invention.

In another preferred embodiment, the expression vector is selected from the group consisting of DNA, RNA, a viral vector, a plasmid, a transposon, other gene transfer system, and a combination thereof.

Preferably, the expression vector comprises a viral vector, such as a lentivirus, an adenovirus, an AAV virus, a retrovirus, or a combination thereof.

In the thirteenth aspect of the present invention, it provides a host cell comprising the expression vector of the twelfth aspect of the present invention, or having the polynucleotide of the eleventh aspect of the present invention integrated into the genome.

In another preferred embodiment, the host cell comprises a prokaryotic cell or a eukaryotic cell.

In another preferred embodiment, the host cell is selected from the group consisting of *Escherichia coli,* a yeast cell, a mammalian cell, a bacteriophage, and a combination thereof.

In another preferred embodiment, the prokaryotic cell is selected from the group consisting of *Escherichia coli, Bacillus subtilis, Lactobacillus, Streptomyces, Proteus mirabilis,* and a combination thereof.

In another preferred embodiment, the eukaryotic cell is selected from the group consisting of *Pichia pastoris, Saccharomyces cerevisiae, Schizosaccharomyces fission,* Trichoderma, and a combination thereof.

In another preferred embodiment, the eukaryotic cell is selected from the group consisting of insect cells such as fall armyworm, plant cells such as tobacco, BHK cells, CHO cells, COS cells, myeloma cells, and combinations thereof.

In another preference embodiment, the host cell is preferably mammalian cells, more preferably HEK293 cells, CHO cells, BHK cells, NSO cells, or COS cells.

In another preferred embodiment, the host cell is *Pichia pastoris.*

In the fourteenth aspect of the present invention, it provides a method for producing an anti-Trop2 single domain antibody or Fc fusion protein thereof, which comprises the steps:
(a) culturing the host cell of the thirteenth aspect of the present invention under conditions suitable for producing the single domain antibody or Fc fusion protein thereof, thereby obtaining a culture containing the anti-Trop2 single domain antibody or Fc fusion protein thereof;
(b) isolating or recovering the anti-Trop2 single domain antibody or Fc fusion protein thereof from the culture; and
(c) optionally, purifying and/or modifying the anti-Trop2 single domain antibody or Fc fusion protein thereof obtained in step (b).

In the fifteenth aspect of the present invention, it provides an immunoconjugate comprising:
(a) the VHH chain of the anti-Trop2 single domain antibody of the second or fourth aspect of the present invention, the anti-Trop2 single domain antibody of the third or fifth aspect of the present invention, the fusion protein of the eighth aspect of the present invention, the multisepecific antibody of the ninth aspect of the present invention, or the bivalent antibody of the tenth aspect of the present invention. and
(b) a coupling moiety selected from the group consisting of a detectable label, a drug, a toxin, a cytokine, a radionuclide, an enzyme, a gold nanoparticle/nanorod, a nanomagnetic particle, a viral coat protein or VLP, and a combination thereof.

In another preferred embodiment, the radionuclide comprises:
(i) a diagnostic isotope, which is selected from the group consisting of Tc-99m, Ga-68, F-18, I-123, 1-125, I-131, In-111, Ga-67, Cu-64, Zr-89, C-11, Lu-177, Re-188, and a combination thereof; and/or
(ii) a therapeutic isotope, which is selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133 Yb-169, Yb-177, and a combination thereof.

In another preferred embodiment, the coupling moiety is a drug or a toxin.

In another preferred embodiment, the drug is a cytotoxic drug.

In another preferred embodiment, the cytotoxic drug is selected from the group consisting of an anti-tubulin drug, a DNA minor groove binding agent, a DNA replication inhibitor, an alkylating agent, an antibiotic, a folate antagonist, an antimetabolite, a chemotherapeutics sensitizer, a topoisomerase inhibitor, vinca alkaloid, and a combination thereof.

In another preferred embodiment, examples of particularly useful cytotoxic drug comprises, for example, a DNA minor groove binding reagent, a DNA alkylation reagent, and a tubulin inhibitor, and a typical cytotoxic drug includes, for example, auristatins, camptothecins, duocarmycins, etoposides, maytansines and maytansinoids (such as DM1 and DM4), taxanes, benzodiazepines or benzodiazepine containing drugs (e.g., pyrrolo [1,4] benzodiazepines (PBDs), indolinobenzodiazepines and oxazolidinobenzodiazepines), vinca alkaloids, or a combination thereof.

In another preferred embodiment, the toxin is selected from the group consisting of: auristatins (e.g, auristatin E, auristatin F, MMAE and MMAF), chlortetracycline, maytansoid, ricin, ricin A-chain, combretastatin, docamicin, dolastatin, doxorubicin, daunorubicin, paclitaxel, cisplatin, cc1065, ethidium bromide, mitomycin, etoposide, tenoposide, vincristine, vinblastine, colchicine, dihydroxyanthraxdione, actinomycin, diphtheria toxin, pseudomonas exotoxin (PE) A, PE40, acacia toxin, acacia toxin A chain, capsule root toxin A chain, α-octococcus, white tree toxin, mitogellin, retstrictocin, phenomycin, enomycin, curicin, crotin, calicheamicin, Sapaonaria officinalis inhibitor, glucocorticoids, and a combination thereof.

In another preferred embodiment, the coupling moiety is a detectable label.

In another preferred embodiment, the coupling moiety is selected from the group consisting of: a fluorescent or luminescent label, a radioactive label, MRI (magnetic resonance imaging) or CT (electronic computer X-ray tomography technique) contrast agent, an enzyme capable of producing detectable products, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-diaphorase (DTD) or biphenyl hydrolase-like protein (BPHL)), and a nanoparticle in any form.

In another preferred embodiment, the immunoconjugate comprises a multivalent (e.g., bivalent or quadvalent) VHH chain of the anti-Trop2 single domain antibody of the second or fourth aspect of the present invention, the anti-Trop2 single domain antibody of the third or fifth aspect of the present invention, or the fusion protein of the eighth aspect of the present invention.

In another preferred embodiment, the multivalent refers to comprise a plurality of replicates of the VHH chain of the anti-Trop2 single domain antibody of the second or fourth aspect of the present invention, the anti-Trop2 single domain antibody of the third or fifth aspect of the present invention, or the fusion protein of the eighth aspect of the present invention in the amino acid sequence of the immunoconjugate.

In the sixteenth aspect of the present invention, it provides an antibody-drug conjugate or the pharmaceutically acceptable salt thereof, wherein the structure of the antibody-drug conjugate is shown in formula III:

Ab-(J-U)n (III)

wherein,
Ab is an anti-Trop2 antibody;
U is a drug;
J is a chemical bond or linker.
n is a positive integer;
"-" is a chemical bond or a linker.

In another preferred embodiment, the Trop2 is either a human Trop2 or a Trop2 of a non-human mammal (such as camel Trop2).

In another preferred embodiment, the Ab is anti-Trop2 antibody or the derived antibody thereof.

In another preferred embodiment, the derived antibody is a modification of an anti-TROP2 antibody, including, but not limited to, linking the Trop2 antibody to an Fc fragment, human serum albumin, a polypeptide specifically bound to serum albumin, and polyethylene glycol (PEG), to form a bivalent antibody and/or a multivalent antibody.

In another preferred embodiment, the antibody includes a humanized antibody, a camel antibody, a chimeric antibody.

In another preferred embodiment, the Ab comprises the VHH chain of the anti-Trop2 single domain antibody of the second or fourth aspect of the present invention, the anti-Trop2 single domain antibody of the third or fifth aspect of the present invention, the multispecific antibody of the ninth aspect of the present invention, or the bivalent antibody of the tenth aspect of the present invention.

In another preferred embodiment, the sequence of Ab comprises an amino acid sequence with at least 80%, preferably at least 90%, more preferably at least 95%, and even more preferably at least 99% sequence similarity to any one of the amino acid sequences set forth in SEQ ID NO: 1, 9, or 14.

In another preferred embodiment, the sequence of Ab comprises an amino acid sequence with at least 80%, preferably at least 90%, more preferably at least 95%, and even more preferably at least 99% sequence similarity to the amino acid sequences set forth in SEQ ID NO: 16.

In another preferred embodiment, the amino acid sequence of the Ab is as set forth in SEQ ID NO: 16.

In another preferred embodiment, the drug is connected to the terminal amino group or side chain amino group of the heavy chain constant region or heavy chain variable domain of the anti-Trop2 antibody.

In another preferred embodiment, the drug is connected to the C-terminal amino group of the anti-Trop2 antibody.

In another preferred embodiment, the drug is site-specifically and/or randomly attached to the anti-Trop2 antibody (i.e., in Formula III, the U is site-specifically and/or randomly attached to Ab).

In another preferred embodiment, the U is site-specifically attached to Ab.

In another preferred embodiment, the J is a linker, comprising CC; (G)ₙC, wherein n is an integer from 0 to 4; (A)ₙC, wherein n is an integer from 1 to 4; (G)ₙCG, wherein n is an integer from 0 to 4; (G)ₙSC, wherein n is an integer from 0 to 4; (GGGGS)ₙC, wherein n is an integer from 0 to 4; C(GGGGS) ₙ, wherein n is an integer from 0 to 4; (GGGS)ₙC, wherein n is an integer from 0 to 4; C(GGGS)ₙ, wherein n is an integer from 0 to 4; (GGS)ₙC, wherein n is an integer from 0 to 4, C(GGS)ₙ, wherein n is an integer from 0 to 4; GSCC; CDV; VDC; LPTEG; GGGGCGGGG; (G)ₙCA, wherein n is an integer from 0 to 4; (A)ₙCA, wherein n is an integer from 1 to 4; (G)ₙCGA, wherein n is an integer from 0 to 4; GGGGCGGGGA; MPA-AEEA, Val-Cit-PABC, polyethylene glycol (PEG), or a combination thereof.

In another preferred embodiment, the linker comprises derivatives of MPA-AEEA, MPA-AEEA Val-Cit-PABC, or polyethylene glycol (PEG), including but not limited to substitution, modification, or deletion of one or more functional groups on their respective basis.

In another preferred embodiment, the linker is a combination of GGC and Val-Cit-PABC.

In another preferred embodiment, the degree of polymerization of chemical bonds is a positive integer greater than or equal to 1.

In another preferred embodiment, the drug includes a cytotoxic drug, an immunomodulator, an inhibitor for enzyme and hormone.

In another preferred embodiment, the drug comprises monomethylaurestatin E (MMAE), monomethylaurestatin F (MMAF), Eribulin, exatecan, maytansine, SN-38, etc.

In the seventeenth aspect of the present invention, it provides a pharmaceutical composition containing:
(i) the VHH chain of the anti-Trop2 single domain antibody of the second or fourth aspect of the present invention, the anti-Trop2 single domain antibody of the third or fifth aspect of the present invention, the fusion protein of the eighth aspect of the present invention, the multisepecific antibody of the ninth aspect of the present invention, the bivalent antibody of the tenth aspect of the present invention, the immunoconjugate of the fifteenth aspect of the present invention, and/or the antibody-drug conjugate of the sixteenth aspect of the present invention; and
(ii) a pharmaceutically acceptable carrier.

In another preferred embodiment, the pharmaceutical composition is an injection.

In another preferred embodiment, the pharmaceutical composition is used for the preparation of a drug for preventing and treating Trop2-related diseases or conditions.

In another preferred embodiment, the Trop2-related disease or condition is cancer or tumor.

In another preferred embodiment, the cancer or tumor includes but is not limited to: gastric cancer, cervical cancer, breast cancer, lung cancer, prostate cancer, colon cancer, uterine papillary serous carcinoma, ovarian cancer, glioblastoma, medulloblastoma, urothelial cancer, head and neck cancer, renal cancer, Kaposi's sarcoma, pancreatic cancer, etc.

In an eighteenth aspect of the present invention, it provides a use of the VHH chain of the anti-Trop2 antibody of the second or fourth aspect of the present invention, the anti-Trop2 single domain antibody of the third or fifth aspect of the present invention, the fusion protein of the eighth aspect of the present invention, the multisepecific antibody of the ninth aspect of the present invention, the bivalent antibody of the tenth aspect of the present invention, the immunoconjugate of the fifteenth aspect of the present invention, or the antibody-drug conjugate of the sixteenth aspect of the present invention, for the preparation of:
(a) drugs used for the prevention and/or treatment of Trop2-related diseases or conditions;
(b) a detection reagent, detection plate, or detection kit used for the detection of human Trop2 molecules.

In another preferred embodiment, the Trop2-related disease or condition is cancer or tumor.

In another preferred embodiment, the cancer or tumor includes but is not limited to: gastric cancer, cervical cancer, breast cancer, lung cancer, prostate cancer, colon cancer, uterine papillary serous carcinoma, ovarian cancer, glioblastoma, medulloblastoma, urothelial cancer, head and neck cancer, renal cancer, Kaposi's sarcoma, pancreatic cancer, etc.

In another preferred embodiment, the detection comprises a flow cytometry detection, a cellular immunofluorescence detection, and an ELISA detection.

In another preferred embodiment, the use is diagnostic and/or non-diagnostic, and/or therapeutic and/or non-therapeutic.

In the nineteenth aspect of the present invention, it provides a recombinant protein having:
(a) the VHH chain of the second or fourth aspect of the present invention, the anti-Trop2 single domain antibody of the third or fifth aspect of the present invention, the fusion protein of the eighth aspect of the present invention, the multisepecific antibody of the ninth aspect of the present invention, and/or the bivalent antibody of the tenth aspect of the present invention; and
(ii) optionally a tag sequence assisting expression and/or purification.

In another preferred embodiment, the tag sequence includes an Fc tag, an HA tag and a 6His tag.

In another preferred embodiment, the recombinant protein specifically binds to Trop2 protein.

In the twentieth aspect of the present invention, it provides a Trop2 protein detection reagent comprising:
(i) the VHH chain of the second or fourth aspect of the present invention, the single domain antibody of the third or fifth aspect of the present invention, the fusion protein of the eighth aspect of the present invention, the immunoconjugate of the fifteenth aspect of the present invention, or the recombinant protein of the nineteenth aspect of the present invention; and
(ii) an acceptable carrier for detection.

In another preferred embodiment, the coupling moiety of the immunoconjugate is a diagnostic isotope.

In another preferred embodiment, the acceptable carrier for detection is a non-toxic, inert, aqueous carrier medium.

In another preferred embodiment, the detection reagent is one or more reagents selected from the group consisting of an isotope tracer, a contrast agent, a flow cytometry detection reagent, a cellular immunofluorescence detection reagent, a magnetic nanoparticle and an imaging agent.

In another preferred embodiment, the detection reagent is used for *in vivo* or *in vitro* detection.

In another preferred embodiment, the dosage form of the detection reagent is liquid or powder (e.g., aqua, injection, lyophilized powder, tablet, buccal, inhaler).

In the twenty-first aspect of the present invention, it provides a kit for detecting Trop2 protein, which comprises the detection reagent of the twentieth aspect of the present invention, and instructions.

In another preferred embodiment, the instructions describe that the kit is used for non-invasively detecting the Trop2 expression of the subject to be tested.

In the twenty-second aspect of the present invention, it provides a method of treating the Trop2-related diseases or conditions, which comprises a step of: administering the VHH chain of the second or fourth aspect of the present invention, the single domain antibody of the third or fifth aspect of the present invention, the fusion protein of the eighth aspect of the present invention, the multispecific antibody of the ninth aspect of the present invention, the bivalent antibody of the tenth aspect of the present invention, the immunoconjugate of the fifteenth aspect of the present invention, and/or the antibody-drug conjugate of the sixteenth aspect of the present invention, or the pharmaceutical composition of the seventeenth aspect of the present invention to a subject in need thereof.

In another preferred embodiment, the subject comprises a mammal, such as a human.

In the twenty-third aspect of the present invention, it provides a method for detecting Trop2 protein in a sample, which comprises the steps:
(1) contacting the VHH chain of the second or fourth aspect of the present invention, the single domain antibody of the third or fifth aspect of the present invention, the fusion protein of the eighth aspect of the present invention, or the immunoconjugate of the fifteenth aspect of the present invention with a sample; and
(2) detecting the formation of an antigen-antibody complex, wherein the formation of a complex indicates the presence of Trop2 protein in the sample.

In another preferred embodiment, the method is an *in vitro* method.

In another preferred embodiment, the method is a non-diagnostic and non-therapeutic method.

It should be understood that within the scope of the present invention, each technical features of the present invention described above and in the following (such as examples) may be combined with each other to form a new or preferred technical solution, which is not listed here due to space limitations.

### DESCRIPTION OF THE DRAWINGS

FIG.1 shows the results of the binding activity of Trop2 single domain antibodies to A431 cells detected by FACS.
FIG.2 shows the results of the binding activity of humanized Trop2 single domain antibodies to A431 cells detected by FACS.
FIG.3 shows the results of the binding activity of humanized Trop2 single domain antibodies to BxPC3 cells detected by FACS.
FIG.4 shows the results of the endocytosis activity of humanized Trop2 single domain antibodies in BxPC3 cells detected by FACS.
FIG.5 shows the results of the binding activity of long-acting humanized Trop2 single domain antibody-drug conjugate to A431 cells detected by FACS.
FIG.6 shows the results of the binding activity of long-acting humanized Trop2 single domain antibody-drug conjugate to BxPC3 cells detected by FACS.
FIG.7 shows the results of the killing effect of long-acting humanized Trop2 single domain antibody-drug conjugate on A431 cells detected by CCK8.
FIG.8 shows the results of the killing effect of long-acting humanized Trop2 single domain antibody-drug on BxPC3 cells detected by CCK8.
FIG.9 shows the results of the anti-tumor effect of long-acting humanized Trop2 single domain antibody-drug conjugate on BxPC3 tumor mice.

### DETAILED DESCRIPTION

Through extensive and deep research, after a large number of of screening, the inventor suprisingly obtained an anti-Trop2 single domain antibody for the first time. The experimental results show that the anti-Trop2 single domain antibody can specifically recognize Trop2 and has good binding specificity and affinity. The long-acting anti-Trop2 antibody formed in tandem by the anti-Trop2 single domain antibody and the anti-serum albumin single domain antibody of the present invention can further conjugate with drugs to form an antibody-drug conjugate, which can effectively kill the tumor cells expressing Trop2 *in vivo or in vitro.* On this basis, the present invention was completed.

### Term

In order to make this disclosure easier to understand, the following terms are defined.

Unless otherwise specified, all singular terms also include the plural, active tense, and past tense of the term.

Unless otherwise explicitly stated in the context, the term "approximately" includes values within the standard deviation range of the value.

Unless otherwise explicitly stated in the context, the words such as "comprise", "have", "include" should be understood as having an inclusive meaning throughout the entire specification and claims, rather than an exclusive or exhaustive meaning; That is to say, the meaning of "including but not limited to". Unless otherwise specified, "comprise" includes "consist of...".

According to the present invention, "subjects" or "patients" are animals, including human patients who require anti-cancer treatment. In certain aspects, the present invention can also be applied in veterinary practice to any mammal or other animal that requires such Trop2 targeted anticancer therapy. This may include, for example, non-human primates, dogs, cats, pigs, horses, and any other animals targeted to Trop2 for anti-cancer treatment.

As used herein, the terms "the single domain antibody of the present invention", "the single domain antibody of the present invention", "the anti-Trop2 single domain antibody of the present invention", "Trop2 single domain antibody of the present invention", "anti-Trop2 single domain antibody" and "Trop2 single domain antibody" have the same meaning and can be used interchangeably to refer to a single domain antibody that specifically recognizes and binds to Trop2, including human Trop2.

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy chain and each light chain. Each heavy chain has a variable region (VH) at one end, followed by a plurality of constant regions. Each light chain has a variable region (VL) at one end and a constant region at the other end; the constant region of light chain is opposite to the first constant region of heavy chain, and the variable region of light chain is opposite to the variable region of heavy chain. Special amino acid residues form an interface between the variable regions of a light chain and a heavy chain.

As used herein, the terms "single domain antibody(sdAb)", "VHH", "nanobody" have the same meaning and can be used interchangeably to refer to cloning the variable region of the heavy chain of the antibody, constructing a single domain antibody (VHH) composed of only one heavy chain variable region, which is the smallest antigen-binding fragment with complete function. Usually, the antibody with natural deletion of light chain and heavy chain constant region 1(CH1) is obtained firstly, and then the variable region of the antibody heavy chain is cloned to construct a single domain antibody (VHH) composed of only one heavy chain variable region.

As used herein, the term "variable" means that certain portion of the variable region in an antibody differs in sequence, which is responsible for the binding and specificity of various specific antibodies to their specific antigen. However, the variability is not distributed evenly throughout the variable regions of an antibody. It is concentrated in three fragments called complementarity determining regions (CDRs) or hypervariable regions in light chain and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partical β-sheet structure. The CDRs in each chain are closely linked together via the FR regions, and form the antigen binding site of an antibody together with the CDRs of the other chain (see Kabat et al., NIH Publ. No. 91-3242, Volume I, pages 647-669 (1991)). Constant regions are not directly involved in the binding of antibodies to antigen, however, they exhibit different effector functions, such as participating in the antibody-dependent cytotoxicity of antibodies.

As known to those skilled in the art, immunoconjugates and the fusion expression products include: conjugates formed by connecting drugs, toxins, cytokines, radionuclides, enzymes or other diagnostic or therapeutic molecules to the antibody or fragment thereof of the present invention.

As used herein, the terms "heavy chain variable region" and "VH" can be used interchangeably.

As used herein, the terms "variable region" and "complementarity determining region (CDR)" can be used interchangeably.

In a preferred embodiment of the present invention, the heavy chain variable region of the antibody comprises three complementarity determining regions, CDR1, CDR2, and CDR3.

In a preferred embodiment of the present invention, the heavy chain of the antibody comprises the above-mentioned heavy chain variable region and the heavy chain constant region.

In the present invention, the terms "antibody of the present invention", "protein of the present invention", or "polypeptide of the present invention" may be used interchangeably and refer to a polypeptide that specifically binds to Trop2 protein, such as a protein or polypeptide having a heavy chain variable region. They may contain or do not contain initial methionine.

The present invention also provides other proteins or fusion expression products having the antibody of the present invention. Specifically, the present invention includes any protein or protein conjugate and fusion expression product (i.e., immunoconjugate and fusion expression product) having a heavy chain containing a variable region, as long as the variable region is the same as or has at least 90% homology, preferably at least 95% homology with the variable region of the heavy chain of the antibody of the present invention.

In general, the antigen binding characteristics of an antibody can be described by three specific regions located in the heavy chain variable region, called the complementarity determining regions (CDRs), which separate the fragment into four frame regions (FRs). The amino acid sequence of the four FRs is relatively conservative and does not directly participate in the binding reaction. These CDRs form a loop structure, and spatially close to each other through the β-sheet formed by the FRs between them. The CDRs on the heavy chain and the CDRs on the corresponding light chain constitute the antigen-binding site of the antibody. The amino acid sequences of antibodies of the same type can be compared to determine which amino acids constitute the FR or CDR regions.

The variable regions of the heavy chain of the antibody of the present invention are of particular interest because at least part of them involve binding to antigens. Therefore, the present invention includes those molecules with a CDR-bearing antibody heavy chain variable region, as long as their CDRs have more than 90% (preferably more than 95%, most preferably more than 98%) homology with the CDRs identified here.

The present invention includes not only intact antibodies, but also fragments of immunologically active antibodies or fusion proteins formed by antibodies with other sequences. Thus, the present invention also includes fragments, derivatives and analogs of the antibody.

As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or sequence used to purify the polypeptide or proteinogen sequence, or a fusion protein formed with a 6His tag). According to the teachings herein, these fragments, derivatives and analogs are within the scope of well-known to those skilled in the art.

The antibody of the present invention refers to a polypeptide having Trop2 binding activity and comprising the above-mentioned CDR regions. The term also includes variant forms of polypeptides comprising the CDR regions described above that have the same function as the antibody of the present invention. These variants include (but are not limited to): deletion, insertion and/or substitution of one or more (usually 1-50, preferably 1-30, more preferably 1-20, most preferably 1-10) amino acids, and addition of one or more (usually within 20, preferably within 10, more preferably within 5) amino acids at the C-terminus and/or N-terminus. For example, in the art, substitution with amino acids of similar properties generally does not alter the function of the protein. For another example, addition of one or more amino acids to the C-terminus and/or N-terminus usually does not alter the function of the protein. The term also includes active fragments and active derivatives of the antibody of the present invention.

The variant forms of the polypeptide include homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

The present invention also provides other polypeptides, such as fusion proteins containing single domain antibodies or fragments thereof. In addition to the almost full-length polypeptide, the present invention also includes fragments of the single domain antibody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "conservative variant of the antibody of the present invention" refers to a polypeptide formed by substituting at most 10, preferably at most 8, more preferably at most 5, and most preferably at most 3 amino acids with amino acids of the same or similar properties as compared with the amino acid sequence of the antibody of the present invention. These conservative variant polypeptides are best produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the above antibody or fragment thereof or fusion protein thereof. The polynucleotide of the present invention may be in the form of DNA or RNA. DNA form includes cDNA, genomic DNA, or synthetic DNA. DNA may be single stranded or double stranded. DNA may be a coding strand or a non-coding strand.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that includes sequence encoding the polypeptide, or a polynucleotide that also includes additional coding and/or non-coding sequences.

The present invention also relates to a polynucleotide that hybridize to the above-mentioned sequence and have at least 50%, preferably at least 70%, and more preferably at least 80% identity between the two sequences. In particular, the present invention relates to a polynucleotide that is hybridizable to the polynucleotide of the present invention under strict conditions. In the present invention, "strict conditions" refers: (1) hybridization and elution at lower ionic strength and higher temperature, such as 0.2 × SSC, 0.1% SDS, 60 °C; or (2) hybridiztion with the addition of denaturing agent, such as 50%(v/v) formamide, 0.1% calf serum/0.1% Ficoll, 42 °C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

The full-length nucleotide sequence or fragments of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence can be obtained by first synthesizing multiple small fragments followed by ligation. In addition, the coding sequence of the heavy chain and the expression tag (such as 6His) can be fused together to form a fusion protein.

Once the relevant sequence is obtained, the recombination method can be used to obtain the relevant sequence in large quantities. This is usually to clone it into a vector, then transfer it into a cell, and then separate the relevant sequence from the proliferated host cell by conventional methods. The biomolecules (nucleic acids, proteins, etc.) involved in the present invention include biomolecules in isolated form.

At present, the DNA sequence encoding the protein (or its fragment, or its derivative) of the present invention can be obtained completely by chemical synthesis. The DNA sequence can then be introduced into various existing DNA molecules (or, for example, vectors) and cells known in the art. In addition, mutations can be introduced into the protein sequence of the present invention by chemical synthesis.

The present invention also relates to a vector comprising the appropriate DNA sequence as described above and an appropriate promoter or control sequence. These vectors can be used to transform appropriate host cells to enable them to express proteins.

Host cells may be prokaryotic cells, such as bacterial cells; or lower eukaryotic cells, such as yeast cells; or higher eukaryotic cells, such as mammalian cells. Representative examples include: *Escherichia coli, Streptomyces;* bacterial cells of *Salmonella typhimurium;* fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as *Escherichia coli,* the competent cells capable of absorbing DNA can be harvested after the exponential growth period and treated with CaCl₂, the steps used are well known in the art. Another method is to use MgCl₂. If necessary, the transformation can also be carried out by electroporation. When the host is eukaryotic, the following DNA transfection methods can be used: calcium phosphate co-precipitation method, conventional mechanical methods such as microinjection, electroporation, liposome packaging, etc.

The obtained transformant can be cultured by conventional methods to express the polypeptide encoded by the gene of the present invention. Depending on the host cell used, the medium used in the culture may be selected from a variety of conventional medium. Culture is carried out under conditions suitable for host cell growth. When the host cells grow to an appropriate cell density, the selected promoter is induced by a suitable method (such as temperature conversion or chemical induction), and the cells are cultured for a period of time.

The recombinant polypeptide in the above method may be expressed in the cell, or on the cell membrane, or secreted outside the cell. If necessary, the recombinant protein can be isolated and purified by various separation methods using its physical, chemical and other properties. These methods are well known to those skilled in the art. Examples of these methods include, but are not limited to, conventional renaturation treatment, treatment with a protein precipitant (salting-out method), centrifugation, osmotic breakage, ultra-treatment, ultra-centrifugation, molecular sieve chromatography (gel filtration), adsorption chromatography, ion exchange chromatography, high performance liquid chromatography (HPLC) and other liquid chromatography techniques and combinations of these methods.

The antibody of the present invention can be used alone, or can be combined or coupled with a detectable label (for diagnostic purposes), a therapeutic agent, a PK (protein kinase) modifying moietiy, or any combination of these substances.

A detectable marker for diagnostic purposes includes, but is not limited to, a fluorescent or luminescent label, a radioactive label, a MRI (magnetic resonance imaging) or CT (electronic computer X-ray tomography technique) contrast agent, or an enzyme capable of producing a detectable product.

Therapeutic agents that may bind or conjugate with the antibodies of the invention include but are not limited to: 1. a radionuclide; 2. a biological toxin; 3. a cytokine such as IL-2, etc; 4. a gold nanoparticle/nanorod; 5. a viral particle; 6. a liposome; 7. a nanomagnetic particle; 8. a prodrug-activating enzyme (e.g., DT-diaphorase (DTD) or biphenyl hydrolase-like protein (BPHL)).

### Anti-Trop2 single domain antibody

In the present invention, it provides an anti-Trop2 single domain antibody, and the anti-Trop2 single domain antibody includes monomer, bivalent (bivalent antibody), quadrivalent (quadrivalent antibody), and/or multivalent (multivalent antibody).

In one aspect of the present invention, it provides an anti-Trop2 single domain antibody, and the complementarity determining regions (CDRs) of the VHH chain of the antibody comprise CDR1 as set forth in SEQ ID NO: 6, CDR2 as set forth in SEQ ID NO: 7, and CDR3 as set forth in SEQ ID NO: 8. In another preferred embodiment, the anti-Trop2 domain antibody comprises a VHH chain as set forth in SEQ ID NO: 1 or 9.

In another aspect of the present invention, it provides an anti-Trop2 single domain antibody, and the anti-Trop2 single domain antibody is a humanized antibody and comprises a VHH chain as set forth in SEQ ID NO: 14.

In a preferred embodiment of the present invention, the anti-Trop2 single domain antibody comprises one, two or more the VHH chains having amino acid sequence as set forth in SEQ ID NO: 1 or 9, and/or SEQ ID NO: 14.

In another preferred embodiment, the anti-Trop2 single domain antibody comprises two VHH chains having amino acid sequence as set forth in SEQ ID NO: 1, 9 and/or 14.

In another preferred embodiment, the anti-Trop2 single domain antibody comprises four VHH chains having amino acid sequence as set forth in SEQ ID NO: 1, 9 and/or 14.

In a preferred embodiment of the present invention, two or four VHH chains are linked by a linking peptide.

In another preferred embodiment, the linking peptide is selected from the following sequences: (GₐS_{b})ₓ-(GₘSₙ)_{y}, wherein a, b, m, n, x, y = 0 or 1 or 2 or 3 or 4 or 5 or 6 or 7 or 8 or 9 or 10 (preferably, a = 4 and b = 1, m = 3 and n = 1).

### Labeled single domain antibody

In a preferred embodiment of the present invention, the single domain antibody carries a detectable label. More preferably, the label is selected from the group consisting of an isotope, a colloidal gold label, a colored label or a fluorescent label.

Colloidal gold labeling may be carried out using methods known to those skilled in the art. In a preferred embodiment of the present invention, the single domain antibody against Trop2 is labeled with colloidal gold to obtain a colloidal gold labeled single domain antibody.

### Anti-serum albumin single domain antibody

The present invention also provides an anti-serum albumin single domain antibody, and the VHH chain of the antibody comprises the following complementarity determining regions: CDR1 as set forth in SEQ ID NO: 24, CDR2 as set forth in any one of SEQ ID NO: 25, 29, 30 or 31, and CDR3 as set forth in SEQ ID NO: 26.

In another preferred embodiment, the VHH chain of the antibody comprises CDR1 as set forth in SEQ ID NO: 24, CDR2 as set forth in SEQ ID NO: 25, and CDR3 as set forth in SEQ ID NO: 26; preferably, the amino acid sequence of the VHH chain thereof is as set forth in SEQ ID NO: 15.

In another preferred embodiment, the VHH chain of the antibody comprises CDR1 as set forth in SEQ ID NO: 24, CDR2 as set forth in SEQ ID NO: 25, and CDR3 as set forth in SEQ ID NO: 26; preferably, the amino acid sequence of the VHH chain thereof is as set forth in SEQ ID NO: 34.

In another preferred embodiment, the VHH chain of the antibody comprises CDR1 as set forth in SEQ ID NO: 24, CDR2 as set forth in SEQ ID NO: 30, and CDR3 as set forth in SEQ ID NO: 26; preferably, the amino acid sequence of the VHH chain thereof is as set forth in SEQ ID NO: 35.

In another preferred embodiment, the VHH chain of the antibody comprises CDR1 as set forth in SEQ ID NO: 24, CDR2 as set forth in SEQ ID NO: 31, and CDR3 as set forth in SEQ ID NO: 26; preferably, the amino acid sequence of the VHH chain thereof is as set forth in SEQ ID NO: 36.

### Bivalent antibody

The present invention also provides a bivalent antibody comprising the anti-Trop2 single domain antibody of the present invention, and the structure of the bivalent antibody from N-terminus to C-terminus is as shown in the Formula IIa or IIb:

Ab1-P-Ab2 (IIa);

Ab2-P-Ab1 (IIb);

wherein,
Ab1 is the anti-Trop2 single domain antibody of the present invention;
Ab2 is the other single domain antibody; and
P is none or a flexible linker.

In one embodiment of the present invention, the Ab2 is anti-serum albumin single domain antibody, which is tandem with the anti Trop2 single domain antibody of the present invention, thereby forming the long-acting anti Trop2 single domain antibody of the present invention. In one preferably embodiment of the present invention, the structure of the bivalent antibody from N-terminus to C-terminus is as shown in the Formula IIa, wherein, Ab1 is the anti-Trop2 single domain antibody of the third or fifth aspect of the present invention, Ab2 is the anti-serum albumin single domain antibody of the seventh aspect of the present invention; preferably, the Ab1 has an amino acid sequence as set forth in SEQ ID NO: 9, and Ab2 has an amino acid sequence as set forth in SEQ ID NO: 15, P is a peptide linker and has a structure of (GGGGS)n, where n is a positive integer from 1 to 5.

### Antibody-Drug Conjugate (ADC)

The present invention also provides an antibody-drug conjugate (ADC) based on the antibody according to the present invention.

The term antibody-drug conjugate (ADC) refers to a monoclonal antibody or antibody fragment that is connected to a biologically active toxic drug through a linking unit. The antibodies or antibody fragments described in this disclosure can be coupled to effector molecules in any manner. For example, antibodies or antibody fragments can be attached to toxic drugs through chemical or recombinant means. The chemical methods for preparing fusion or conjugate are known in this field. The method used for coupling antibodies or antibody fragments with drugs must be able to connect antibodies with toxic drugs without interfering with the ability of antibodies or antibody fragments to bind to target molecules.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody. Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc.

Cytotoxic drugs refer to substances that inhibit or prevent cell function and/or cause cell death or destruction. Cytotoxic drugs can generally kill tumor cells at sufficiently high concentrations, but due to their lack of specificity, while killing tumor cells, they can also lead to apoptosis of normal cells, resulting in serious side effects. Cytotoxic drugs include toxins such as small molecule toxins or enzyme-active toxins of bacterial, fungal, plant or animal origin, radioisotopes (e.g., radioisotopes of At²¹¹, I¹³¹, I¹²⁵, Y⁹⁰, Re¹⁸⁶, Re¹⁸⁸, Sm¹⁵³, B1²¹², P³², and Lu), chemotherapy drugs, antibiotics, and ribozymes.

The antibody of the present invention and the cytotoxic drugs may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) can be used to link a variety of functional groups, including imaging agents (such as chromophores and fluorophores), diagnostic agents (such as MRI contrast agents and radioisotopes), stabilizers (such as ethylene glycol polymer) and therapeutic agents. An antibody can be conjugated to a functional agent to form a conjugate of the antibody-functional agent. A functional agent (e.g., a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a jonit (also known as linker) between a drug and an antibody. The terms " linker unit " or " linking fragment " or " linking unit " means chemical structure fragment or bound that one end is linked to an antibody or an antigen-binding fragment thereof, and the other end thereof is linked to a drug, or may be linked to another linker and then linked to a drug. The linker can be a degradable or non-degradable linker. Typically, a degradable linker is easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g., lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine; or a tripeptide, such as glycine-phenylalanine-glycine; or a tetrapeptide, such as glycine-glycine-phenylalanine-glycine. Other suitable degradable linkers include, for example, pH sensitive linkers (e.g., linkers that are hydrolyzed at a pH of below 5.5, such as hydrazone linkers) and linkers that are degraded under reducing conditions (e.g., disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g., iodo-, bromo- or chloro-substituted) amide; a halogenated (e.g., iodo-, bromo- or chloro-substituted) ester; a halogenated (e.g., iodo-, bromo- or chloro-substituted) methyl ketone, a benzyl halide (e.g., iodide, bromide or chloride); vinyl sulfone, pyridyl disulfide; a mercury derivative such as 3,6-di-(mercurymethyl)dioxane, wherein the counter ion is CH₃COO-, Cl- or NO₃-; and polymethylene dimethyl sulfide thiosulfonate. The linker may include, for example, a maleimide linked to an antibody via thiosuccimide.

In the present invention, a drug-linker compound can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), linking an antibody to a drug-linker compound (or linker-drug, LD, such as LD-1~LD17 as shown in the present invention).

In certain embodiments, the method according to the present invention comprises: under conditions sufficient to form an antibody-linker conjugate, linking an antibody to a linker compound. In these embodiments, the method according to the present invention further comprises: under conditions sufficient to covalently link the drug moiety to the antibody via a linker, linking the antibody-linker conjugate to the drug moiety.

Drug loading, also known as Drug-to-Antibody Ratio (DAR), refers to the average number of drugs conjugated to each antibody in ADC. It can be within the range of approximately 1 to about 10 drugs per antibody, and in some embodiments, within the range of approximately 1 to about 8 drugs per antibody, preferably from the ranges of 2-8, 2-7, 2-6, 2-5, 2-4, 3-4, 3-5, 5-6, 5-7, 5-8, and 6-8. For example, the drug loading can be the mean of 1, 2, 3, 4, 5, 6, 7, 8, 9, 10. The ADC general formula disclosed herein includes a collection of antibody-drug conjugates within a certain range as mentioned above. In the embodiments disclosed herein, the drug loading can be represented as n, which is a decimal or integer. Conventional methods such as UV/visible spectroscopy, mass spectrometry, ELISA assay, and HPLC can be used to determine drug loading.

In one embodiment disclosed herein, cytotoxic drugs are coupled to antibodies through linking units.

The loading of ligand drug conjugates can be controlled using the following non limiting methods, including:
(1) Control the molar ratio of drug linker fragments to monoclonal antibodies,
(2) Control reaction time and temperature,
(3) Choose different reaction reagents.

### Pharmaceutical composition

The present invention also provides a composition. Preferably, the composition is a pharmaceutical composition comprising the above-mentioned antibody or active fragment thereof or fusion protein thereof or immunoconjugate thereof and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to) intraperitoneal, intravenous, or topical administration.

The pharmaceutical composition according to the present invention can be directly used for binding to a Trop2 protein molecule, and thus can be used for treating Trop2-related diseases, such as cancer, etc. In addition, other therapeutic agents may be used at the same time.

The pharmaceutical composition according to the present invention comprises a safe and effective amount (e.g., 0.001-99 wt%, preferably 0.01-90 wt%, preferably 0.1-80 wt%) of the antibody above mentioned according to the present invention (or a conjugate thereof) and a pharmaceutically acceptable carrier or excipient. Such carriers include, but are not limited to, saline, buffer, glucose, water, glycerol, ethanol, and a combination thereof. The pharmaceutical formulation should match the mode of administration. The pharmaceutical composition of the present invention may be prepared in the form of an injection, for example, by conventional methods using normal saline or aqueous solutions containing glucose and other adjuvants. The pharmaceutical composition such as an injection and solution should be manufactured under sterile conditions. The dosage of the active ingredient is a therapeutically effective amount, for example, about 10 µg/kg body weight per day to about 50 mg/kg body weight. In addition, the polypeptide according to the present invention may also be used in combination with an additional therapeutic agent.

In one embodiment in the present invention, when a pharmaceutical composition is used, a safe and effective amount of the immunoconjugate of the present invention is administered to a mammal, wherein the safe and effective amount is typically at least about 10 µg/kg body weight, and in most cases no more than about 50 mg/kg body weight, preferably about 10 µg/kg body weight to about 10 mg/kg body weight. Of course, the specific dosage should also consider factors such as the administration route and the patient's health status, which are all within the skill range of a skilled physician.

### Kit

The present invention also provides a kit containing the antibody (or fragment thereof) or the detection reagent of the present invention. In a preferred embodiment of the present invention, the kit further comprises a container, instructions for use, and a buffer, etc.

The present invention also provides a detection kit for detecting the Trop2 level, which comprises an antibody that recognizes Trop2 proteins, a lysis medium for dissolving a sample, common reagents and buffers required for detection, such as various buffers, detection labels, detection substrates, etc. The detection kit may be an *in vitro* diagnostic device.

### Detection method

The present invention also relates to a method for detecting Trop2 protein. The steps of the method are roughly as follows: obtaining a cell and/or tissue sample; dissolving the sample in a medium; and detecting the level of Trop2 protein in the dissolved sample.

In the detection method of the present invention, the sample used is not particularly limited, and a representative example is a cell-containing sample present in a cell preservation solution.

### Application

As described above, the single domain antibody of the present invention has a wide range of biological application value and clinical application value, and its application relates to the diagnosis and treatment of the Trop2-related diseases, basic medical research, biological research and other fields. A preferred application is for clinical diagnosis and targeted therapy of Trop2, such as for cancers or tumors with high Trop2 expression (including, but not limited to: treatment and diagnosis of gastric cancer, cervical cancer, breast cancer, lung cancer, prostate cancer, colon cancer, uterine papillary serous carcinoma, ovarian cancer, glioblastoma, medulloblastoma, urothelial cancer, head and neck cancer, renal cancer, Kaposi's sarcoma, pancreatic cancer, etc.)

### The main advantages of the present invention include:

(1) The Trop2 single domain antibody of the present invention has good cell binding activity.
(2) The Trop2 single domain antibody of the present invention has good endocytosis activity.
(3) The anti-serum albumin single domain antibody provided by the present invention has good binding activity and high expression yield, and the purity and quality uniformity after one-step purification are higher, which is beneficial for the preparation of medicine.
(4) The Trop2 single domain antibody of the present invention can be used to construct a long-acting Trop2 single domain antibody-conjugate drugs. The long-acting Trop2 single domain antibody-conjugate drug has good binding activity with A431 cells and BxPC3 cells, and is not affected by the presence of HSA proteins; Moreover, the long-acting Trop2 single domain antibody-conjugate drug has a significant killing effect on A431 cells and BxPC3 cells, and has significant BxPC3 tumor killing activity, with the high-dose group showing complete tumor regression.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in Sambrook and Russell et al., Molecular Cloning: A Laboratory Manual (third edition) (2001) (CSHL press), or according to the conditions recommended by the manufacturer. Unless otherwise stated, percentages and parts are calculated by weight.

Sequences of the present invention:
**Amino acid sequences (The wavy line portion represents the CDR region, and the underlined portion represents the joint or linker)**
Trop2 Nb1 (SEQ ID NO: 1) 
QVQLQESGGGSVQAGGSLRLSCAASGFTFSRACMGWFRQASGKEREGV
AQVYTGGGSTSYADSVKGRFTISQDNAKNTLYLQMNSLKPEDTAVYYCAVG
QPYGACYYSDLAPADFSYWGQGTQVTVSS
Trop2 Nb1 FR1 (SEQ ID NO: 2)
   QVQLQESGGGSVQAGGSLRLSCAAS
Trop2 Nb1 FR2 (SEQ ID NO: 3)
   WFRQASGKEREGVAQ
Trop2 Nb1 FR3 (SEQ ID NO: 4)
   SYADSVKGRFTISQDNAKNTLYLQMNSLKPEDTAVYYC
Trop2 Nb1 FR4 (SEQ ID NO: 5)
   WGQGTQVTVSS
Trop2 Nb1 CDR1 (SEQ ID NO: 6)
   GFTFSRACMG
Trop2 Nb1 CDR2 (SEQ ID NO: 7)
   VYTGGGST
Trop2 Nb1 CDR3 (SEQ ID NO: 8)
   AVGQPYGACYYSDLAPADFSY
Trop2 HuNb1 (SEQ ID NO: 9) 
EVQLLESGGGLVQPGGSLRLSCAASGFTFSRACMGWFRQASGKGLEGV
AQVYTGGGSTSYADSVKGRFTISQDNAKNTLYLQMNSLKPEDTAVYYCAVG
QPYGACYYSDLAPADFSYWGQGTLVTVSS
Trop2 HuNb1 FR1 (SEQ ID NO: 10)
   EVQLLESGGGLVQPGGSLRLSCAAS
Trop2 HuNb1 FR2 (SEQ ID NO: 11)
   WFRQASGKGLEGVAQ
Trop2 HuNb1 FR3 (SEQ ID NO: 12)
   SYADSVKGRFTISQDNAKNTLYLQMNSLKPEDTAVYYC
Trop2 HuNb1 FR4 (SEQ ID NO: 13)
   WGQGTLVTVSS
Trop2 HuNb2 (SEQ ID NO: 14) 
EVQLLESGGGLVQPGGSLRLSCVDSGSTYSRRYMGWFRRAPGKGLEGV
SVISPYGDSTYYADSVKDRFTISRDNAKNTVYLQMNSLKPEDTAMYYCAAS
WPYQTASLDAMDFTDWGQGTLVTVSS
Alb Nb1 (SEQ ID NO: 15) 
EVQLQESGGGLVEPGGSLRLSCVASGFTFQHYAMSWVRQAPGKGLEW
VSSISLVGGGTSYADSVKGRFTMSRDNAKNTLYLQLNSLKSEDTAMYYCVR
GWHPDVPRPLGQGTLVTVSS
Alb Nb1 CDR1 (SEQ ID NO: 24)
   GFTFQHYAMS
Alb Nb1 CDR2 (SEQ ID NO: 25)
   ISLVGGGT
Alb Nb1 CDR3 (SEQ ID NO: 26)
   VRGWHPDVPRP
HuNb1-Alb Nb1 (SEQ ID NO: 16) 
EVQLLESGGGLVQPGGSLRLSCAASGFTFSRACMGWFRQASGKGLEGV
AQVYTGGGSTSYADSVKGRFTISQDNAKNTLYLQMNSLKPEDTAVYYCAVG
QPYGACYYSDLAPADFSYWGQGTLVTVSSGGGGSGGGGSGGGGSGGGGSE
VQLQESGGGLVEPGGSLRLSCVASGFTFQHYAMSWVRQAPGKGLEWVSSIS
LVGGGTSYADSVKGRFTMSRDNAKNTLYLQLNSLKSEDTAMYYCVRGWHP
DVPRPLGQGTLVTVSS
HuNb1-Alb Nb1-GGC (SEQ ID NO: 17)

EVQLLESGGGLVQPGGSLRLSCAASGFTFSRACMGWFRQASGKGLEGV
AQVYTGGGSTSYADSVKGRFTISQDNAKNTLYLQMNSLKPEDTAVYYCAVG
QPYGACYYSDLAPADFSYWGQGTLVTVSSGGGGSGGGGSGGGGSGGGGSE
VQLQESGGGLVEPGGSLRLSCVASGFTFQHYAMSWVRQAPGKGLEWVSSIS
LVGGGTSYADSVKGRFTMSRDNAKNTLYLQLNSLKSEDTAMYYCVRGWHP
DVPRPLGQGTLVTVSSGGC

### Base sequence

Trop2 Nb1 (SEQ ID NO: 18) 
CAGGTTCAGCTGCAGGAATCTGGTGGTGGTTCTGTTCAGGCGGGTGG
TTCTCTGCGTCTGTCTTGCGCGGCGTCTGGTTTCACCTTCTCTCGTGCGTG
CATGGGTTGGTTCCGTCAGGCGTCTGGTAAAGAACGTGAAGGTGTTGCGC
AGGTTTACACCGGTGGTGGTTCTACCTCTTACGCGGACTCTGTTAAAGGT
CGTTTCACCATCTCTCAGGACAACGCGAAAAACACCCTGTACCTGCAGAT
GAACTCTCTGAAACCGGAAGACACCGCGGTTTACTACTGCGCGGTTGGTC
AGCCGTACGGTGCGTGCTACTACTCTGACCTGGCGCCGGCGGACTTCTCT
TACTGGGGTCAGGGTACCCAGGTTACCGTTTCTTCT
Trop2 HuNb1 (SEQ ID NO: 19) 
GAGGTCCAGCTGCTGGAGAGCGGAGGCGGACTCGTCCAGCCTGGCG
GCTCCCTCCGGCTCTCCTGTGCCGCTTCTGGATTTACATTTAGCAGGGCTT
GTATGGGATGGTTCAGGCAGGCTTCAGGGAAAGGGTTGGAGGGTGTTGC
CCAGGTTTATACCGGAGGGGGCAGCACTTCCTACGCCGATTCAGTCAAAG
GCAGGTTTACCATCTCCCAGGATAATGCTAAGAATACCCTGTATCTCCAG
ATGAACAGTTTGAAACCCGAGGACACAGCTGTGTACTACTGTGCTGTGGG
GCAGCCCTACGGAGCATGCTATTATAGCGATCTCGCCCCAGCAGACTTTT
CATACTGGGGGCAGGGGACCCTCGTTACAGTTTCCTCT
Trop2 HuNb2 (SEQ ID NO: 20) 
GAAGTGCAGCTGCTGGAGAGCGGAGGAGGACTGGTTCAGCCTGGAG
GAAGCCTGAGACTGAGCTGCGTGGACAGCGGAAGCACATACAGCAGAAG
ATACATGGGATGGTTCAGAAGAGCACCTGGAAAAGGCCTGGAGGGGGTG
TCTGTGATCAGCCCTTATGGAGACAGCACCTACTACGCCGATAGCGTGAA
GGACAGATTCACCATAAGCCGGGACAACGCCAAGAACACAGTGTACCTG
CAAATGAACAGCCTGAAGCCCGAAGATACTGCTATGTACTACTGCGCCGC
CAGCTGGCCTTACCAGACAGCTTCACTGGACGCTATGGATTTTACCGATT
GGGGCCAGGGGACCCTGGTTACAGTCTCTAGC
Alb Nb1 (SEQ ID NO: 21) 
GAGGTTCAGTTGCAGGAAAGTGGCGGTGGGCTGGTGGAGCCCGGAG
GTTCCCTCCGCTTGAGTTGCGTCGCCTCTGGTTTTACCTTTCAGCACTATG
CTATGTCATGGGTTCGCCAGGCCCCAGGGAAGGGGCTGGAGTGGGTCAGT
TCTATTTCCCTGGTCGGTGGCGGAACATCCTATGCAGATTCTGTGAAGGG
GCGCTTTACAATGTCCCGCGACAATGCCAAGAACACTTTGTACCTGCAGC
TCAACTCTCTGAAGTCCGAAGATACAGCCATGTATTACTGCGTGCGTGGA
TGGCATCCAGACGTGCCTAGACCCCTCGGCCAGGGTACATTGGTGACAGT
CTCCAGC
HuNb1-Alb Nb1 (SEQ ID NO: 22) 
GAGGTCCAGCTGCTGGAGAGCGGAGGCGGACTCGTCCAGCCTGGCG
GCTCCCTCCGGCTCTCCTGTGCCGCTTCTGGATTTACATTTAGCAGGGCTT
GTATGGGATGGTTCAGGCAGGCTTCAGGGAAAGGGTTGGAGGGTGTTGC
CCAGGTTTATACCGGAGGGGGCAGCACTTCCTACGCCGATTCAGTCAAAG
GCAGGTTTACCATCTCCCAGGATAATGCTAAGAATACCCTGTATCTCCAG
ATGAACAGTTTGAAACCCGAGGACACAGCTGTGTACTACTGTGCTGTGGG
GCAGCCCTACGGAGCATGCTATTATAGCGATCTCGCCCCAGCAGACTTTT
CATACTGGGGGCAGGGGACCCTCGTTACAGTTTCCTCTGGTGGCGGAGGG
TCTGGTGGTGGAGGATCAGGGGGTGGAGGTTCAGGAGGCGGGGGAAGTG
AGGTTCAGTTGCAGGAAAGTGGCGGTGGGCTGGTGGAGCCCGGAGGTTC
CCTCCGCTTGAGTTGCGTCGCCTCTGGTTTTACCTTTCAGCACTATGCTAT
GTCATGGGTTCGCCAGGCCCCAGGGAAGGGGCTGGAGTGGGTCAGTTCTA
TTTCCCTGGTCGGTGGCGGAACATCCTATGCAGATTCTGTGAAGGGGCGC
TTTACAATGTCCCGCGACAATGCCAAGAACACTTTGTACCTGCAGCTCAA
CTCTCTGAAGTCCGAAGATACAGCCATGTATTACTGCGTGCGTGGATGGC
ATCCAGACGTGCCTAGACCCCTCGGCCAGGGTACATTGGTGACAGTCTCC
AGC
HuNb1-Alb Nb1-GGC (SEQ ID NO: 23)

GAGGTCCAGCTGCTGGAGAGCGGAGGCGGACTCGTCCAGCCTGGCG
GCTCCCTCCGGCTCTCCTGTGCCGCTTCTGGATTTACATTTAGCAGGGCTT
GTATGGGATGGTTCAGGCAGGCTTCAGGGAAAGGGTTGGAGGGTGTTGC
CCAGGTTTATACCGGAGGGGGCAGCACTTCCTACGCCGATTCAGTCAAAG
GCAGGTTTACCATCTCCCAGGATAATGCTAAGAATACCCTGTATCTCCAG
ATGAACAGTTTGAAACCCGAGGACACAGCTGTGTACTACTGTGCTGTGGG
GCAGCCCTACGGAGCATGCTATTATAGCGATCTCGCCCCAGCAGACTTTT
CATACTGGGGGCAGGGGACCCTCGTTACAGTTTCCTCTGGTGGCGGAGGG
TCTGGTGGTGGAGGATCAGGGGGTGGAGGTTCAGGAGGCGGGGGAAGTG
AGGTTCAGTTGCAGGAAAGTGGCGGTGGGCTGGTGGAGCCCGGAGGTTC
CCTCCGCTTGAGTTGCGTCGCCTCTGGTTTTACCTTTCAGCACTATGCTAT
GTCATGGGTTCGCCAGGCCCCAGGGAAGGGGCTGGAGTGGGTCAGTTCTA
TTTCCCTGGTCGGTGGCGGAACATCCTATGCAGATTCTGTGAAGGGGCGC

TTTACAATGTCCCGCGACAATGCCAAGAACACTTTGTACCTGCAGCTCAA
CTCTCTGAAGTCCGAAGATACAGCCATGTATTACTGCGTGCGTGGATGGC
ATCCAGACGTGCCTAGACCCCTCGGCCAGGGTACATTGGTGACAGTCTCC
AGCGGGGGCTGC

### Example 1: Screening and identification of TROP2-targeting single domain antibodies

One Xinjiang Bactrian camel was immunized with high-purity hTrop2 (ECD)-Fc protein. After 7 times of immunization, total RNA was isolated from the camel's peripheral blood, and the VHH gene was amplified by reverse transcription and PCR. The VHH gene was cloned onto the phage vector pMECS and transformed into a TG1 host cell to construct a phage display single domain antibody library. After 3-4 rounds of "bind-wash-elute" screening, Trop2 specific phages were obtained. Then 600 clones were randomly selected for identification by PE-ELISA, and all the positive clones were identified by sequencing, and then candidate single domain antibodies were expressed and purified from *E.coli* strains.

### Example 2: Binding activity of candidate Trop2 antibodies to A431 cells

Flow cytometry was used to detect the binding activity of these candidate single domain antibodies to Trop2 high expression cell line A431. After the cultured A431 cells were digested with trypsin and neutralized in complete medium, the cells were washed once with PBS, collected and counted, and the cells were added to a 96-well V-bottom plate, 2E5 cells/well. Diluted candidate single domain antibodies and control antibodies BMK1, BMK2 and BMK3 (control antibody sequences are all referred to patent CN111518212B, where BMK1 sequence is the amino acid sequence as set forth in SEQ ID NO: 78, BMK2 sequence is the amino acid sequence as set forth in SEQ ID NO: 65, and BMK3 sequence is the amino acid sequence as set forth in SEQ ID NO: 68) were added to the plate respectively and incubated at 4°C for 40min. After centrifugation, cells were washed with PBS twice, and APC anti-HA antibody was added to incubate at 4°C for 40min. After centrifugation, cells were washed with PBS twice, the supernatant was removed, and 200µL/well PBS was added to resuspend cells. APC signal of each sample was detected by flow cytometry.

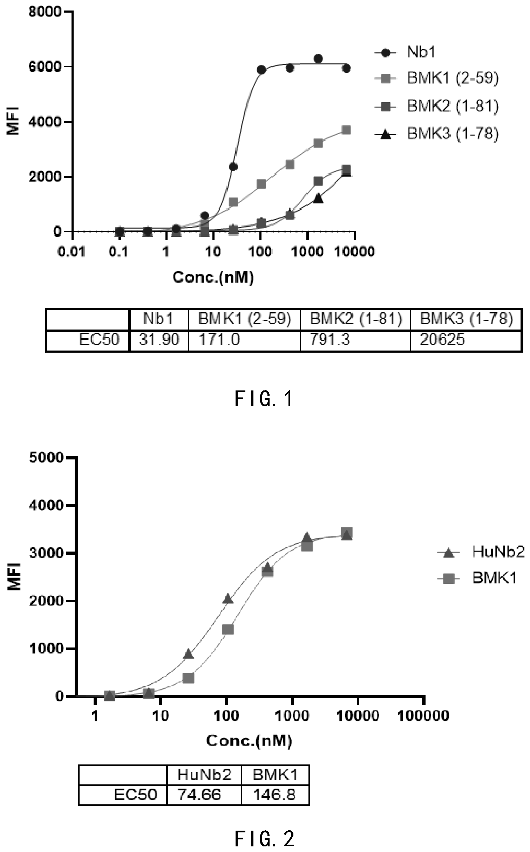

As shown in FIG.1, candidate antibody Nb1 has a significant advantage in A431 cell binding activity compared with the other three control antibodies.

### Example 3: Humanization of Trop2 single domain antibody

Humanization was performed on the candidate antibody Nb1, and the humanization method is described in Example 4 of Patent CN2018101517526. The modified antibody sequence is set forth in SEQ ID NO: 9 (marked as HuNb1).

In addition, the amino acid sequence set forth in SEQ ID NO: 47 in patent CN111518212B has been re-humanized, and the modified antibody sequence is set forth in SEQ ID NO: 14 (marked as HuNb2). The binding activity of humanized antibodies was detected again using flow cytometry, and the detection method is identical to that described in Example 2 above. As shown in FIG.2, the humanized Trop2 single domain antibody HuNb2 exhibits better cell binding activity compared to the original humanized antibody (SEQ ID NO: 78 disclosed in CN111518212B, i.e., BMK1 in FIG.2).

### Example 4: Binding activity of humanized Trop2 single domain antibodies to BxPC3 cells

Flow cytometry was used to detect the binding activity of humanized Trop2 antibodies prepared to Trop2 high expression cell line BxPC3. The detection method is identical to that described in Example 2. Cultured BxPC3 cells and diluted antibodies were incubated at 4 °C for 40 minutes. APC anti-HA antibody was added to the cells washed with PBS and incubated at 4°C for 40min. After centrifugation, cells were washed and the supernatant was removed. APC signal of each sample was detected by flow cytometry after PBS suspension.

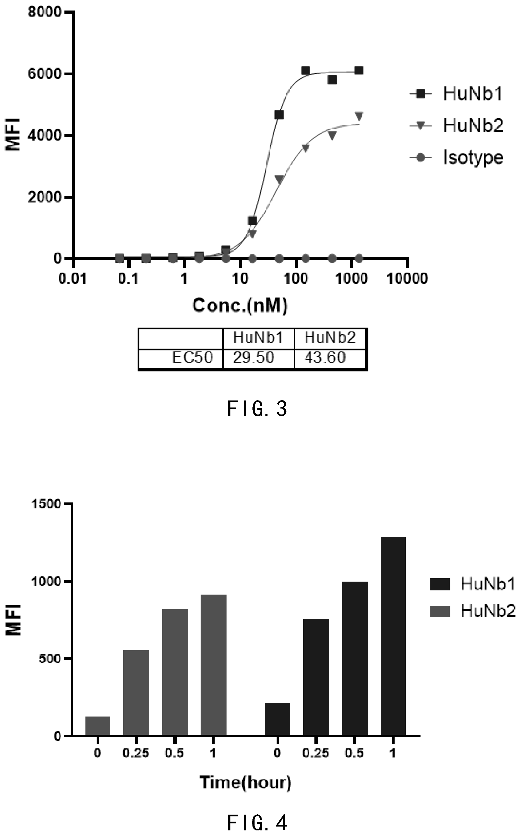

As shown in FIG.3, humanized Trop2 single domain antibodies HuNb1 and HuNb2 exhibit good BxPC3 cell binding activity.

### Example 5: Identification of endocytosis activity of humanized Trop2 single domain antibody

The cultured A431 and BXPC-3 cells were collected and counted, and then divided into U-bottom plates. The diluted single domain antibodies were added to the cells and incubated at 4°C for 30 minutes. APC anti-HA antibody was added to cells washed with PBS and incubated at 4°C for 30min. After washing the cells, the medium containing 1%FBS was added to the non-endocytosis (0h) group and the maximum endocytosis (0h) group, and the cells were suspended and placed at 4°C. At the same time, the medium containing 1%FBS was added to the endocytosis groups (0.25h, 0.5h and 1h) and placed in a CO₂ incubator at 37°C. The corresponding samples were taken out and placed in a 4°C refrigerator at 0.25h, 0.5h and 1h, respectively. Finally, the supernatant was removed by centrifugation, then the Stripping buffer was added and incubated at 4°C. After washing the cells, PBS was added to resuspend the cells and the APC signal of each sample was detected by flow cytometry.

As shown in FIG.4, both the humanized Trop2 single domain antibodies HuNb1 and HuNb2 have good endocytotic activity.

### Example 6: Screening of anti-serum albumin single domain antibody

The half-life of Trop2 single domain antibody was prolonged by using the albumin single domain antibody sequence HuNb3-11 and its optimized mutant sequence disclosed in patent CN2022113943304. *Pichia Pastoris* was used to express HuNb3-11 and its mutant (CDR2 with single amino acid site mutation), and the vector construction method and protein expression method were referred to example 4 of patent CN202110164376.6.

The HuNb3-11 sequence is shown below, where the underlined portion is CDR:

EVQLQESGGGLVEPGGSLRLSCVASGFTFQHYAMSWVRQAPGKGLEW
VSSISMVGGGTSYADSVKGRFTMSRDNAKNTLYLQLNSLKSEDTAMYYCVR
GWHPDVPRPLGQGTLVTVSS (SEQ ID NO: 27)

**Table 1 Comparison of HuNb3-11 and mutant CDR sequences**

| Antibody No. | CDR1 | CDR2 | CDR3 |
|---|---|---|---|
| HuNb3-11 | GFTFQHYAMS (SEQ ID NO: 24) | ISMVGGGT (SEQ ID NO: 28) | VRGWHPDVPRP (SEQ ID NO: 26) |
| Alb Nb1 | Same as HuNb3-11 | ISLVGGGT (SEQ ID NO: 25) | Same as HuNb3-11 |
| Alb Nb2 | Same as HuNb3-11 | ISVVGGGT (SEQ ID NO: 29) | Same as HuNb3-11 |
| Alb Nb3 | Same as HuNb3-11 | ISIVGGGT (SEQ ID NO: 30) | Same as HuNb3-11 |
| Alb Nb4 | Same as HuNb3-11 | ISTVGGGT (SEQ ID NO: 31) | Same as HuNb3-11 |
| Alb Nb5 | Same as HuNb3-11 | ISGVGGGT (SEQ ID NO: 32) | Same as HuNb3-11 |
| Alb Nb6 | Same as HuNb3-11 | ISYVGGGT (SEQ ID NO: 33) | Same as HuNb3-11 |

**Table 2 Expression yield and binding activity of anti-serum albumin single domain antibody**

| **Antibody No.** | **Expression yield of Pichia pastoris** | **Relative binding activity EC50** | **CEX main peak purity** |
|---|---|---|---|
| HuNb3-11 | 3 g/L | / | 81% |
| Alb Nb1 | 4 g/L | 92% | 93% |
| Alb Nb2 | 3g/L | 95% | 92% |
| Alb Nb3 | 2.5 g/L | 80% | 89% |
| Alb Nb4 | 2.5 g/L | 71% | 92% |
| Alb Nb5 | 3 g/L | 10% | / |
| Alb Nb6 | 4 g/L | 0% | / |

The results show that the HuNb3-11 mutants expressed in yeast have a higher expression yield, higher purity and better quality and uniformity after one-step purification, and maintain its original binding activity (Table 2).

### Example 7: Construction and expression of long-acting Trop2 antibody and conjugated drugs thereof

The candidate Trop2 single domain antibody HuNb1 was constructed in tandem with an albumin single domain antibody (Alb Nb1, SEQ ID NO: 15) to form a bivalent with long-acting function. And the linker sequence (e.g., GG) and free cysteine (C) were designed at its C-terminus for site-specific coupling of load drugs. The amino acid sequence of the constructed long-acting Trop2 single domain antibody conjugated drug is as set forth in SEQ ID NO: 17. The optimized base sequence of the amino acid sequence (SEQ ID NO: 23) according to Pichia Pastoris codon was cloned into pPICZaA, then it was linearized and electrically transformed into X33 receptor cells to construct a stable expression cell line. Subsequently, the long-acting Trop2 antibody HuNb1-Alb Nb1-GGC was purified from the supernatant of the induced culture of the cell line.

Using the same method, the candidate Trop2 single domain antibody HuNb1 was linked in tandem with albumin single domain antibodies Alb Nb2 (SEQ ID NO: 34), Alb Nb3 (SEQ ID NO: 35), and Alb Nb4 (SEQ ID NO: 36) to construct long-acting Trop2 single domain antibodies HuNb1-Alb Nb2-GGC, HuNb1-Alb Nb3-GGC, and HuNb1-Alb Nb4-GGC. The yeast expression yield of the four antibodies is shown in Table 3. It can be seen that HuNb1-Alb Nb1-GGC has the highest expression yield.

**Table 3 Yeast expression yield of long-acting Trop2 single domain antibodies**

| **Antibody No.** | **Yeast expression yield** |
|---|---|
| HuNb1-Alb Nb1-GGC | 2.0g/L |
| HuNb 1-Alb Nb2-GGC | 2.0g/L |
| HuNb1-Alb Nb3-GGC | 1.5g/L |
| HuNb1-Alb Nb4-GGC | 1.8 g/L |

Based on the above data, the purified long-acting Trop2 antibody HuNb1-Alb Nb1-GGC was subjected to a reduction treatment of 10mM TCEP at 4 °C for 16 hours. The excess TCEP was filtered out, and then the 2-fold molar amount of vc-PAB-MMAE (Monomethylauristatin E) was added and incubated at room temperature for 1 hour. An appropriate amount of acetylcysteine was added to terminate the reaction, and subjected to ultrafiltration to change the solution to PBS for activity analysis. The long-acting Trop2 antibody conjugated drug prepared is called HuNb1-Alb Nb1-MMAE.

### Example 8: Identification of cell binding activity of long-acting Trop2 antibody conjugated drugs

The long-acting Trop2 antibody conjugated drug HuNb1-Alb Nb1-MMAE was incubated with A431 and BxPC3 cells, and the binding activity of the antibody conjugated drug to the cells was detected by flow cytometry. The detection method is identical to that described in Example 2. Cultured A431 cells and BxPC3 cells were incubated with the diluted antibody at 4°C for 40min (human blood albumin was added during incubation). The cells were washed with PBS and then added with goat anti-VHH antibody and incubated at 4°C for 40 min. The cells were washed and then added with Donkey-anti-Goat antibody (Alexa Fluor488) and incubated at 4°C for 40 min. After centrifugation, the cells were washed and the supernatant was removed, and the cells were resuspended in PBS and then the Alexa Fluor488 signal of each sample was detected by flow cytometry.

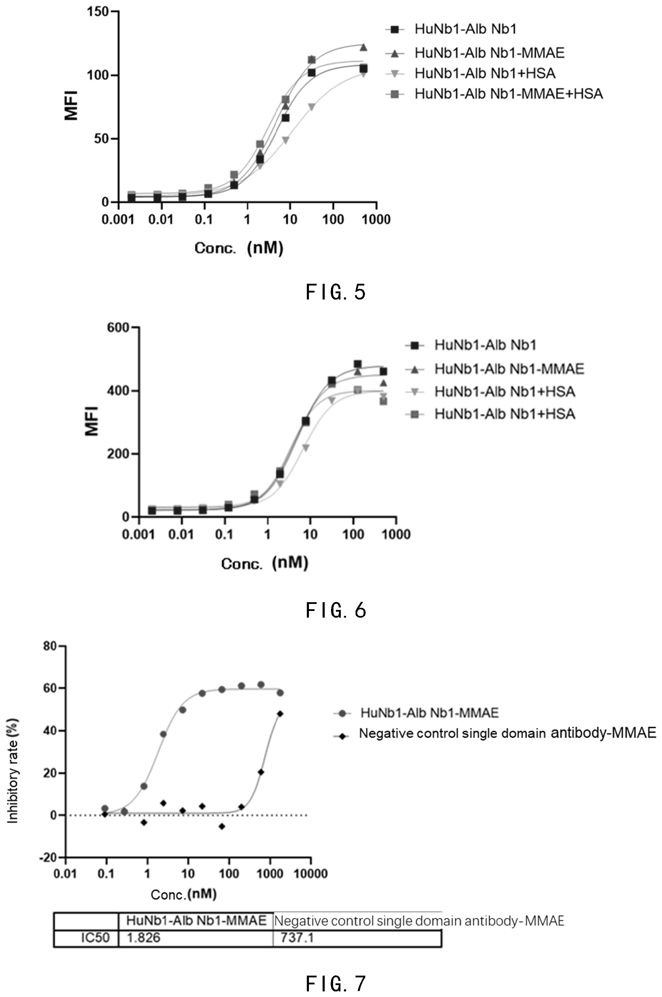

As shown in FIG.5 and FIG.6, the long-acting Trop2 antibody conjugated drug HuNb1 Alb Nb1-MMAE exhibits good binding activity to A431 cells and BxPC3 cells, and is not affected by the presence of HSA protein.

### Example 9: Identification of cell killing activity of long-acting Trop2 antibody conjugated drugs

The long-acting Trop2 single domain antibody conjugated drug HuNb1-Alb NF1-MMAE was gradiently diluted in cell medium and incubated with human serum albumin for 20 minutes, then co-incubated with A431 cells and BxPC3 cells, respectively. Cell Counting Kit-8 was added after 96 hours of culture at 37°C in 5% CO₂ incubator, and the cells were incubated in the incubator for 2.5 hours. After reading the data (OD450) in the microplate reader, IC50 was calculated.

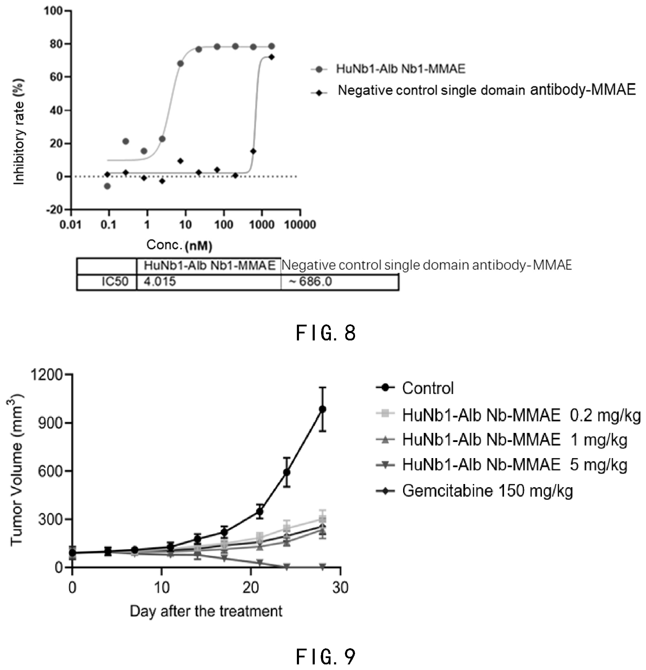

As shown in FIG.7 and FIG.8, the long-acting Trop2 single domain antibody conjugated drug HuNb1-Alb Nb1-MMAE shows significant killing effect on both A431 cells and BxPC3 cells.

### Example 10: Identification of anti-tumor activity of long-acting Trop2 antibody conjugated drugs

NCG mice were inoculated with BxPC3 pancreatic cancer cells for tumorigenesis. When the tumors grew to about 80mm³, they were divided into groups, with 6 mice in each group. Three dose groups were set up to be intraperitoneally injected with HuNb1-Alb Nb1-MMAE at 0.2mg/kg, 1mg/kg and 5mg/kg, three times a week, respectively. The negative control group was intraperitoneally injected with PBS. The positive control group was intraperitoneally injected with 150mg/kg Gemcitabine, three times a week.

As shown in FIG.9, the long-acting Trop2 antibody conjugated drug HuNb1-Alb Nb1-MMAE has significant BXPC3 tumor killing activity, wherein the tumor of high-dose group completely fades after treatment for 20 days, which is significantly better than the treatment effect of Gemcitabine.

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. A VHH chain of an anti-TROP2 single domain antibody, wherein the VHH chain comprises framework regions (FRs) and complementarity determining regions (CDRs), and the complementarity determining regions (CDRs) of the VHH chain comprise CDR1 as set forth in SEQ ID NO: 6, CDR2 as set forth in SEQ ID NO: 7, and CDR3 as set forth in SEQ ID NO: 8.

2. The VHH chain of claim 1, wherein the amino acid sequence of the VHH chain of the anti-Trop2 single domain antibody is set forth in SEQ ID NO: 1 or 9.

3. A anti-Trop2 single domain antibody, wherein the single domain antibody comprises the VHH chain of claim 1.

4. A VHH chain of a humanized anti-TROP2 single domain antibody, wherein the amino acid sequence of the VHH chain is set forth in SEQ ID NO: 14.

5. An anti-Trop2 single domain antibody, wherein the antibody comprises the VHH chain of claim 4.

6. A VHH chain of an anti-serum albumin single domain antibody, wherein the complementarity determining regions (CDRs) of the VHH chain comprises CDR1 as set forth in SEQ ID NO: 24, CDR2 as set forth in any one of SEQ ID NO: 25, 29, 30 or 31, and CDR3 as set forth in SEQ ID NO: 26.

7. An anti-serum albumin single domain antibody, wherein the antibody comprises the VHH chain of claim 6.

8. An anti-Trop2 single domain antibody Fc fusion protein, wherein the structure of the fusion protein from N-terminus to C-terminus is shown in the Formula Ia or Ib:
A-L-B (Ia);
B-L-A (Ib);
wherein,
A is the anti-Trop2 single domain antibody of claim 3 or claim 5;
B is an Fc fragment of IgG; and
L is none or a flexible linker.

9. A multispecific antibody, wherein the multispecific antibody comprises the VHH chain of the anti-Trop2 single domain antibody of claim 1 or 4, the anti-Trop2 single domain antibody of claim 3 or 5, and/or the fusion protein of claim 8.

10. A bivalent antibody, wherein the bivalent antibody comprises the VHH chain of the anti-Trop2 single domain antibody of claim 1 or 4, the anti-Trop2 single domain antibody of claim 3 or 5, or the fusion protein of claim 8.

11. The bivalent antibody of claim 10, wherein the structure of the bivalent antibody from the N-terminus to the C-terminus is shown in Formula IIa or IIb:
Ab1-P-Ab2 (IIa);
Ab2-P-Ab1 (IIb);
wherein,
Ab1 comprises the VHH chain of the anti-Trop2 single domain antibody of claim 1 or 4, or the anti-Trop2 single domain antibody of claim 3 or 5;
Ab2 is the other single domain antibody, preferably, the Ab2 comprises the VHH chain of anti-serum albumin single domain antibody of claim 6, or the anti-serum albumin single domain antibody of claim 7; and
P is none or a flexible linker.

12. The bivalent antibody of claim 11, wherein the Ab1 has the amino acid sequence as set forth in SEQ ID NO: 9, and Ab2 has the amino acid sequence as set forth in SEQ ID NO: 15.

13. A polynucleotide, wherein the polynucleotide encodes a protein selected from the group consisting of the VHH chain of the anti-Trop2 single domain antibody of claim 1 or 4, the anti-Trop2 single domain antibody of claim 3 or 5, the fusion protein of claim 8, the multispecific antibody of claim 9, and the bivalent antibody of claim 10.

14. An expression vector comprising the polynucleotide of claim 13.

15. A host cell comprising the expression vector of claim 14, or having the polynucleotide of claim 13 integrated into the genome.

16. An immunoconjugate containing:
(a) the VHH chain of the anti-Trop2 single domain antibody of claim 1 or 4, the anti-Trop2 single domain antibody of claim 3 or 5, the fusion protein of claim 8, the multispecific antibody of claim 9, or the bivalent antibody of claim 10; and
(b) a coupling moiety selected from the group consisting of a detectable label, a drug, a toxin, a cytokine, a radionuclide, an enzyme, a gold nanoparticle/nanorod, a nanomagnetic particle, a viral coat protein or VLP, and a combination thereof.

17. An antibody-drug conjugate or the pharmaceutically acceptable salt thereof, wherein the structure of the antibody-drug conjugate is shown in Formula III:
Ab-(J-U)n (III)
wherein,
Ab is an anti-Trop2 antibody, and comprises the VHH chain of the anti-Trop2 single domain antibody of claim 1 or 4, the anti-Trop2 single domain antibody of claim 3 or 5, the fusion protein of claim 8, the multispecific antibody of claim 9, or the bivalent antibody of claim 10;
U is a drug;
J is a chemical bond or linker.
n is a positive integer;
"-" is a chemical bond or a linker.

18. A pharmaceutical composition containing:
(i) the VHH chain of the anti-Trop2 single domain antibody of claim 1 or 4, the anti-Trop2 single domain antibody of claim 3 or 5, the fusion protein of claim 8, the multispecific antibody of claim 9, the bivalent antibody of claim 10, the immunoconjugate of claim 16, and/or the antibody-drug conjugate of claim 17; and
(ii) a pharmaceutically acceptable carrier.

19. Use of the VHH chain of the anti-Trop2 single domain antibody of claim 1 or 4, the anti-Trop2 single domain antibody of claim 3 or 5, the fusion protein of claim 8, the multispecific antibody of claim 9, the bivalent antibody of claim 10, the immunoconjugate of claim 16, or the antibody-drug conjugate of claim 17, for the preparation of:
(a) drugs used for the prevention and/or treatment of Trop2-related diseases or conditions;
(b) a detection reagent, detection plate, or detection kit used for detecting human Trop2 molecules.

20. A method of treating Trop2-related diseases or conditions comprising: administering the VHH chain of the anti-Trop2 single domain antibody of claim 1 or 4, the anti-Trop2 single domain antibody of claim 3 or 5, the fusion protein of claim 8, the multispecific antibody of claim 9, the bivalent antibody of claim 10, the immunoconjugate of claim 16, or the antibody-drug conjugate of claim 17, or the pharmaceutical composition of claim 18 to a subject in need thereof.
